# FASCICULE DE BREVET EUROPEEN

(11) **EP 4 301 243 B1**
(45) Date de publication et mention de la délivrance du brevet: **02.07.2025**
(21) Numéro de dépôt: 22708999.2
(22) Date de dépôt: 15.02.2022
(51) Int. Cl.: A61B 17/04

(54) **ANCRE DE SUTURE POUR UN ANCRAGE D'UN TISSU MOU SUR UN OS**
NAHTANKER ZUR VERANKERUNG VON WEICHGEWEBE AN KNOCHEN
SUTURE ANCHOR FOR ANCHORING SOFT TISSUE TO BONE

(30) Priorité: 02.03.2021 FR 2102033
(43) Date de publication de la demande: 10.01.2024
(73) Titulaire: RS4, 26000 Valence (FR)
(72) Inventeur: BATAILLE, Jean-François, 30650 ROCHEFORT DU GARD (FR); BELLUMORE, Yves, 31600 MURET (FR); BOUDARD, Guillaume, 25170 RECOLOGNE (FR); GADEA, François, 83000 TOULON (FR); GEORGE, Thierry, 45160 OLIVET (FR); GUIGNAND, Didier, 69370 SAINT DIDIER AU MONT D'OR (FR); LE RUE, Olivier, 59800 LILLE (FR); MOREEL, Philippe, 13710 FUVEAU (FR); NAVEZ, Grégory, 26000 VALENCE (FR); SARRAN, Olivier, 64140 BILLERE (FR); TOURNIER, Clément, 33000 BORDEAUX (FR)
(74) Mandataire: Germain Maureau
(86) Numéro de dépôt international: PCT/FR2022/050275
(87) Numéro de publication internationale: WO 2022/184989

(56) Documents cités:
- US-A1- 2018 360 438
- US-A1- 2020 155 140

## Description

L'invention se rapporte à une ancre de suture pour un ancrage d'un tissu mou sur un os.

L'invention vise plus particulièrement à fournir une ancre de suture permettant la fixation d'au moins un fil de suture sur un os, un tel fil de suture étant prévu pour être relié à un tissu mou pour amarrer le tissu mou sur sa zone d'amarrage osseuse native (aussi appelée zone d'insertion osseuse) ; étant entendu par « tissu mou » toute partie d'un corps humain ou animal formant un tissu de soutien extra-squelettique servant à supporter une structure ou un organe, et en particulier un ligament, un tendon et un muscle.

Une application favorite, et non limitative, concerne l'ancrage des tendons de la coiffe des rotateurs de l'épaule sur leur zone d'amarrage osseuse native autour de la tête humérale, étant noté que d'autres applications sont envisageables dans l'ancrage de tendon ou de ligament au niveau d'autres articulations telles que la hanche, le genou, le coude, la cheville ou le poignet.

Dans le domaine de la coiffe des rotateurs de l'épaule, il est classique d'opérer sous arthroscopie selon une technique dite de double rang consistant à :
- ancrer une première rangée d'ancres de suture dites de premier rang qui assurent un ancrage et un blocage solide de fils de suture qui serviront à suturer et à amarrer les tendons sur leur zone d'amarrage osseuse native, puis
- ancrer une seconde rangée d'ancres de suture dites de second rang, en position latérale par rapport à la première rangée, qui servent à fixer sur l'os les parties libres des brins des fils de suture et ainsi les tendre pour plaquer les tendons contre la zone d'amarrage osseuse native et donc favoriser la cicatrisation.

Selon les pratiques des chirurgiens et selon la rangée, deux réalisations sont envisageables pour la mobilité du fil de suture procurée par l'ancrage :
- première réalisation : le fil de suture est ancré dans l'os tout en conservant une mobilité, autrement dit le fil de suture peut coulisser en tirant sur l'un ou l'autre de ses brins libres, ce qui est avantageux pour avoir un ajustement du fil de suture ;
- seconde réalisation : le fil de suture est ancré dans l'os et reste fixe, autrement dit le fil de suture ne peut pas coulisser et reste statique quand bien même on tirerait sur l'un ou l'autre de ses brins libres, ce qui est avantageux pour avoir une mise en tension sur le tissu mou.

De manière classique, il est connu d'avoir soit des ancres qui répondent à la première réalisation, soit d'avoir des ancres qui répondent à la seconde réalisation.

A titre d'exemple, les documents US2018/0271514A1, US10639026B2 et FR2925287A1 proposent des ancres qui répondent à la première réalisation en comprenant une partie distale présentant un chas de passage d'un fil de suture, et une partie proximale présentant un filetage qui, une fois l'ancre insérée dans le trou osseux, va bloquer le fil d'ancrage contre la paroi interne du trou osseux. Le document US2018/360438 décrit également un tel type d'ancre, réalisée en une seule pièce, et comprenant sur sa partie distale un chas de passage d'un fil de suture, à l'intérieur duquel est prévu un pont pour pouvoir former une boucle du fil de suture autour du pont.

Le document AU2007202269B2 propose quant à lui un modèle d'ancre répondant à la première réalisation et un autre modèle d'ancre répondant à la seconde réalisation.

L'état de la technique peut également être illustré par les enseignements du document US2020/155140 qui décrit une ancre de suture selon le préambule de la revendication 1, et dans laquelle est prévue une fente, de sorte qu'un fil de suture intérieur puisse être remonté et coincé en haut de la fente, une telle ancre étant prévue pour répondre à la seconde réalisation avec le fil de suture intérieur statique.

L'invention propose quant à elle une ancre de suture qui soit conformée pour répondre à la fois à la première réalisation et à la seconde réalisation, et donc qui puisse être employée à la fois en première rang et en seconde rang, ce qui permet d'avoir un unique modèle d'ancre qui soit adapté pour répondre à toutes les pratiques chirurgicales, en permettant soit un ancrage d'au moins un fil de suture avec mobilité (première réalisation uniquement), soit un ancrage d'au moins un fil de suture sans mobilité (seconde réalisation uniquement), soit un ancrage conjoint d'au moins un fil de suture avec mobilité et d'au moins un fil de suture sans mobilité (première et seconde réalisations combinées).

Un autre but de l'invention est d'offrir une ancre de suture adaptée pour présenter un risque réduit de casse lors d'une insertion par impaction.

Un autre but de l'invention est de réduire les risques de rupture du fil de suture lors d'une insertion par impaction.

A cette fin, l'invention propose une ancre de suture, pour un ancrage d'un tissu mou sur un os, une telle ancre de suture comprenant un premier élément et second élément indépendant du premier élément.

Selon l'invention, le premier élément comprend les caractéristiques suivantes :
- il présente le long d'un axe longitudinal une première extrémité distale et une première extrémité proximale opposées,
- il présente successivement, depuis la première extrémité distale jusqu'à la première extrémité proximale, une section distale d'insertion, une section centrale d'ancrage et une section proximale d'accouplement,
- cette section centrale d'ancrage présente extérieurement et le long de l'axe longitudinal une succession de crans périphériques d'ancrage,
- au moins une rainure longitudinale s'étend le long de l'axe longitudinal et est ménagée à travers et le long de la succession de crans périphériques d'ancrage,
- un trou transversal est ménagé dans cette rainure longitudinale et traverse la section centrale d'ancrage pour former un chas de passage de fil de suture,
- un pont est prévu à l'intérieur du trou transversal pour séparer deux passages décalés l'un de l'autre selon l'axe longitudinal,
- un premier orifice longitudinal est ménagé intérieurement dans la section proximale d'accouplement et dans la section centrale d'ancrage le long de l'axe longitudinal, ce premier orifice longitudinal débouchant sur la première extrémité proximale et débouchant sur le trou transversal en face du pont.

Selon l'invention, le second élément comprend les caractéristiques suivantes :
- il présente une seconde extrémité proximale et une seconde extrémité distale opposées,
- il présente extérieurement au moins un relief d'ancrage,
- un second orifice longitudinal est ménagé intérieurement dans le second élément et débouche sur la seconde extrémité proximale et sur la seconde extrémité distale, ce second orifice longitudinal étant conformé pour pouvoir engager le second élément sur la section proximale d'accouplement du premier élément avec ladite section proximale d'accouplement à l'intérieur dudit second orifice longitudinal.

Ainsi, le premier élément est prévu pour être inséré à l'intérieur d'un trou osseux ménagé dans l'os, soit à la suite d'un perçage préalable soit directement par impaction. Le second élément vient dans un deuxième temps s'accoupler au premier élément, à l'intérieur du trou osseux, pour verrouiller l'ancrage ; cet accouplement s'opérant par engagement du second élément sur la section proximale d'accouplement.

L'ancre de suture est donc configurable dans deux configurations :
- une configuration initiale dans laquelle le premier élément et le second élément sont désaccouplés, avec le second élément éloigné de la section proximale d'accouplement ; et
- une configuration finale (qui correspond à la configuration à la fin de l'opération d'ancrage) dans laquelle le premier élément et le second élément sont accouplés, avec le second élément engagé sur la section proximale d'accouplement.

Il est aussi à noter que la section proximale d'accouplement permet avantageusement de guider le second élément lors de son insertion.

Le premier élément peut embarquer un ou plusieurs fils de suture avec deux possibilités :
- première possibilité : au moins un fil de suture intérieur passe dans le second orifice longitudinal du second élément et dans le premier orifice longitudinal du premier élément pour déboucher dans le trou transversal et présenter une boucle autour du pont, ce fil de suture intérieur ayant deux brins libres qui dépassent de la seconde extrémité proximale du second élément ;
- seconde possibilité : un fil de suture extérieur passe dans l'au moins une rainure longitudinale, sans passer dessus les crans périphériques d'ancrage, pour pénétrer dans le trou transversal et présenter une boucle autour du pont, ce fil de suture extérieur ayant deux brins libres qui dépassent de la première extrémité proximale du premier élément, en sortie de l'au moins une rainure longitudinale, pour être, en situation (autrement dit dans la configuration finale à la fin de l'opération d'ancrage), bloqués par l'au moins un relief d'ancrage prévu sur l'extérieur du second élément.

L'au moins une rainure longitudinale forme une sorte de couloir qui permet au fil de suture extérieur de ne pas être coincé entre les crans périphériques d'ancrage et la paroi interne du trou osseux durant l'insertion du premier élément dans ce trou osseux, en particulier lorsque ce premier élément est inséré par impaction. Ainsi, à la fin de l'insertion de ce premier élément, le fil de suture extérieur demeure n'a pas été endommagé par l'insertion, et il peut être libre de coulisser. Ensuite, le second élément, lorsqu'il va venir s'accoupler au premier élément, va venir coincer le fil de suture extérieur entre le relief d'ancrage et la paroi interne du trou osseux. Donc à la fin de l'opération d'ancrage, ce fil de suture extérieur est bloqué et il ne peut pas coulisser, autrement dit il est statique ce qui correspond à la seconde réalisation précédemment évoquée.

Concernant le fil de suture interne, ce dernier reste mobile à l'intérieur du second orifice longitudinal et du premier orifice longitudinal, pendant et après l'opération d'ancrage, ce qui correspond à la première réalisation précédemment évoquée.

Selon une caractéristique, la succession de crans périphériques d'ancrage comprend au moins un cran proximal, celui situé le plus proche de la première extrémité proximale, et un cran distal, celui situé le plus proche de la première extrémité distale, et l'au moins une rainure longitudinale s'étend au moins depuis le cran proximal jusqu'au cran distal.

Selon une possibilité, le trou transversal est ménagé au moins partiellement au niveau du cran distal, au début donc de la succession de crans périphériques d'ancrage.

Selon une autre possibilité, deux rainures longitudinales sont ménagées à travers et le long de la succession de crans périphériques d'ancrage, les deux rainures longitudinales étant diamétralement opposées vis-à-vis de l'axe longitudinal de sorte que le trou transversal débouche dans les deux rainures longitudinales.

Ainsi, le fil de suture extérieur peut soit avoir ses deux brins qui passent dans la même rainure longitudinale, soit avoir un brin dans l'une des deux rainures longitudinales et un autre brin dans l'autre des deux rainures longitudinales. Il est aussi possible d'avoir un fil de suture extérieur qui passe dans l'une des deux rainures longitudinales et un autre fil de suture extérieur qui passe dans l'autre des deux rainures longitudinales.

Selon une autre possibilité, l'au moins une rainure longitudinale est prolongée, au niveau de la section distale d'insertion et dans la continuité du trou transversal, par une rampe en surélévation.

Une telle rampe est avantageuse pour assurer un libre passage du ou des fils de suture extérieur dans la ou les rainures longitudinales.

Dans une réalisation particulière, la première extrémité distale est en forme de pointe, permettant une insertion par impaction.

Avantageusement, les crans périphériques d'ancrage sont des crans unidirectionnels, c'est-à-dire des crans asymétriques présentant une face inclinée d'attaque tournée en direction de l'extrémité distale.

Dans un mode de réalisation particulier, le second orifice longitudinal est de section non circulaire, et par exemple de section polygonale comme une section hexagonale.

Une telle section non circulaire est avantageuse pour permettre à un instrument d'insertion de coopérer avec ce second orifice longitudinal afin de faire tourner le second élément, par exemple pour un vissage.

Selon une possibilité, le second orifice longitudinal et la section proximale d'accouplement sont conformés pour que le second élément soit libre en rotation autour de l'axe longitudinal lorsque ledit second élément est engagé sur la section proximale d'accouplement.

Selon une autre possibilité, l'au moins un relief d'ancrage prévu extérieurement sur le second élément est un filetage.

Ce filetage peut être avec un pas de vis évolutif pour permettre un verrouillage progressif du second élément.

Selon une caractéristique, le premier orifice longitudinal est de section circulaire, permettant notamment à un instrument d'insertion de tourner dans ce premier orifice longitudinal.

Selon une autre caractéristique, le premier orifice longitudinal présente un épaulement interne, ce qui est avantageux pour permettre à instrument d'insertion de prendre appui sur cet épaulement interne, donc au fond du premier orifice longitudinal du premier élément, réduisant les risques de casse lors d'une insertion par impaction du premier élément.

Dans une réalisation avantageuse, les crans périphériques d'ancrage présentent un premier diamètre externe d'ancrage, et l'au moins un relief d'ancrage présente un second diamètre externe d'ancrage qui est supérieur au premier diamètre externe d'ancrage.

L'invention se rapporte également à un kit de suture, pour un ancrage d'un tissu mou sur un os, ce kit de suture comprenant une ancre de suture selon l'invention, et au moins un fil de suture formant une boucle sur le pont prévu dans le trou transversal.

Selon une première possibilité, précédemment évoquée, l'au moins un fil de suture comprend un fil de suture intérieur qui passe dans le second orifice longitudinal du second élément et dans le premier orifice longitudinal du premier élément pour déboucher dans le trou transversal et présenter une boucle autour du pont, ledit fil de suture intérieur ayant deux brins libres qui dépassent de la seconde extrémité proximale du second élément.

Selon une seconde possibilité, précédemment évoquée, l'au moins un fil de suture comprend un fil de suture extérieur qui passe dans l'au moins une rainure longitudinale, sans passer dessus les crans périphériques d'ancrage, pour pénétrer dans le trou transversal et présenter une boucle autour du pont, ledit fil de suture extérieur ayant deux brins libres qui dépassent de la première extrémité proximale du premier élément, en sortie de l'au moins une rainure longitudinale, pour être, en situation, bloqués par l'au moins un relief d'ancrage prévu sur l'extérieur du second élément.

L'invention concerne aussi l'emploi dans le kit de suture d'un instrument d'insertion propre à insérer par impaction le premier élément de l'ancre de suture, cet instrument d'insertion présentant un orifice interne longitudinal autorisant le passage d'au moins un fil de suture (en l'occurrence un fil de suture intérieur) et présentant une partie distale engagée à l'intérieur du premier orifice longitudinal du premier élément.

Selon une possibilité, le premier orifice longitudinal présente un épaulement interne et la partie distale de l'instrument d'insertion présente une extrémité distale venant en appui sur cet épaulement interne, où la partie distale dépasse de la première extrémité proximale du premier élément de sorte que cet instrument d'insertion ne vient pas en appui contre ladite première extrémité proximale.

Un tel appui de l'instrument d'insertion sur cet épaulement interne, et pas sur la première extrémité proximale du premier élément, permet avantageusement de réduire le risque de casse du premier élément lors de son insertion par impaction.

Selon une autre possibilité, la partie distale de l'instrument d'insertion est libre en rotation autour de l'axe longitudinal à l'intérieur du premier orifice longitudinal.

Dans un mode de réalisation particulier, l'instrument d'insertion présente, dans le prolongement de la partie distale, une partie proximale autour de laquelle est monté coulissant le second élément, cette partie proximale traversant le second orifice longitudinal.

Dans une réalisation particulière, le second orifice longitudinal est de section non circulaire, et par exemple de section polygonale comme une section hexagonale, et la partie proximale de l'instrument d'insertion est de section complémentaire à celle du second orifice longitudinal, de sorte que le second élément est solidaire en rotation de l'instrument d'insertion autour de l'axe longitudinal.

Ainsi, cette partie proximale va permettre de faire tourner le second élément lors de son insertion, ce qui est pratique en particulier si ce second élément présente un filetage qui nécessite une insertion par vissage dans le trou osseux.

D'autres caractéristiques et avantages de la présente invention apparaîtront à la lecture de la description détaillée ci-après, d'un exemple de mise en œuvre non limitatif, faite en référence aux figures annexées dans lesquelles :
[Fig 1] est une vue schématique de côté (à gauche) et en perspective (à droite) d'une ancre de suture selon l'invention en configuration initiale ;
[Fig 2] est une vue schématique de dessus du premier élément (à gauche) et du second élément (à droite) de l'ancre de suture de la Figure 1 ;
[Fig 3] est une vue schématique de côté (à gauche) et en perspective (à droite) de l'ancre de suture de la Figure 1 en configuration finale ;
[Fig 4] est une vue schématique en coupe longitudinale de l'ancre de suture de la Figure 1 en configuration initiale (à gauche) et en configuration finale (à droite) ;

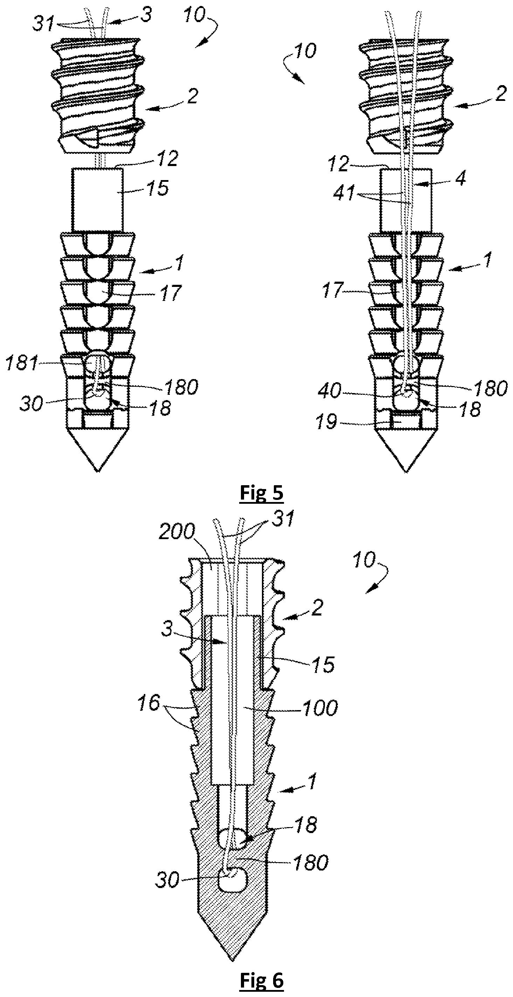

[Fig 5] est une vue schématique de côté de l'ancre de suture de la Figure 1 en configuration initiale avec un fil de suture intérieur (à gauche) et avec un fil de suture extérieur (à droite) ;
[Fig 6] est une vue schématique en coupe longitudinale de l'ancre de suture de la Figure 1 en configuration finale avec un fil de suture intérieur ;
[Fig 7] est une vue schématique de côté (à gauche) et en coupe longitudinale (à droite) de l'ancre de suture de la Figure 1 en configuration initiale, l'ancre de suture étant montée sur un instrument d'insertion d'un kit de suture selon l'invention ;
[Fig 8] est une vue schématique en coupe longitudinale de l'ancre de suture de la Figure 1 en configuration finale avec un fil de suture intérieur, l'ancre de suture étant montée sur l'instrument d'insertion de la Figure 7 ;
[Fig 9] est une vue schématique de côté de l'ancre de suture de la Figure 1 en en configuration initiale avec un fil de suture extérieur (à gauche) et avec à la fois un fil de suture extérieur et un fil de suture intérieur (à droite), l'ancre de suture étant montée sur l'instrument d'insertion de la Figure 7 et étant en partie insérée à l'intérieur d'un trou osseux ménagé dans un os.

En référence aux Figures, une ancre de suture 10 selon l'invention comprend deux éléments 1, 2, à savoir un premier élément 1 et un second élément 2 aptes à coopérer ensemble pour ancrer un ou plusieurs fils de suture 3, 4 sur un os 9, à des fins d'ancrage d'un tissu mou sur l'os 9.

Le premier élément 1 présente, le long d'un axe longitudinal X, une première extrémité distale 11 et une première extrémité proximale 12 opposées, où :
- la première extrémité distale 11 est en forme de pointe, et par exemple de pointe tronconique, et forme ainsi une extrémité auto-perforante permettant de ménager un trou 90 dans l'os 9 par impaction ; et
- la première extrémité proximale 12 est une extrémité plate s'étendant selon un plan orthogonal à l'axe longitudinal X.

Le premier élément 1 présente successivement, depuis la première extrémité distale 11 jusqu'à la première extrémité proximale 12 :
- une section distale d'insertion 13, dont une fonction est de guider l'insertion du premier élément 1 et de l'ancre de suture 10 dans l'os 9 ;
- une section centrale d'ancrage 14, dont une fonction est d'ancrer et bloquer le premier élément 1 et l'ancre de suture 10 dans l'os 9 ; et
- une section proximale d'accouplement 15, dont une fonction est de permettre un accouplement avec le second élément 2.

La section distale d'insertion 13 est composée principalement de la première extrémité distale 11 en forme de pointe.

La section centrale d'ancrage 14 présente extérieurement et le long de l'axe longitudinal X une succession de crans périphériques d'ancrage 16, qui sont des crans unidirectionnels de forme tronconique et qui présentent un premier diamètre externe d'ancrage D1. Cette succession de crans périphériques d'ancrage 16 comprend au moins un cran proximal, celui situé le plus proche de la première extrémité proximale 12, et un cran distal, celui situé le plus proche de la première extrémité distale 11, ainsi qu'un ou plusieurs crans intermédiaires entre le cran proximal et le cran distal. Dans l'exemple illustré, la succession de crans périphériques d'ancrage 16 comprend six crans, et donc quatre crans intermédiaires.

Chaque cran périphérique d'ancrage 16 présente ainsi :
- une face distale, tournée vers la première extrémité distale 11, et qui est une face tronconique, dont le diamètre s'élargit en direction de la première extrémité proximale 12 en partant d'un diamètre minimal jusqu'à atteindre le premier diamètre externe d'ancrage D1 ; et
- une face proximale, tournée vers la première extrémité proximale 12, et qui est une face plate (annulaire) s'étendant selon un plan orthogonal à l'axe longitudinal X.

La section centrale d'ancrage 14 présente par ailleurs deux rainures longitudinales 17, diamétralement opposées vis-à-vis de l'axe longitudinal X, qui s'étendent chacune le long de l'axe longitudinal X et qui sont ménagées à travers et le long de la succession de crans périphériques d'ancrage 16. Chaque rainure longitudinale 17 forme ainsi une gorge ou une saignée à travers les crans périphériques d'ancrage 16. Chaque rainure longitudinale 17 s'étend au moins depuis le cran proximal jusqu'au cran distal, sur au moins toute la longueur de la succession de crans périphériques d'ancrage 16.

La section centrale d'ancrage 14 présente une portion non crantée 140, située entre la section distale d'insertion 13 et le cran distal de la succession de crans périphériques d'ancrage 16. Cette portion non crantée 140 présente une forme cylindrique centré sur l'axe longitudinal X, avec une face périphérique ayant un diamètre externe qui est équivalent au diamètre minimal des crans périphériques d'ancrage 16.

La section proximale d'accouplement 15 présente une forme cylindrique centré sur l'axe longitudinal X, avec une face périphérique lisse ayant un diamètre externe qui est inférieur au diamètre minimal des crans périphériques d'ancrage 16, et cette section proximale d'accouplement 15 prolonge le cran proximal jusqu'à la première extrémité proximale 12.

Sur le premier élément 1, un trou transversal 18 est ménagé dans et à travers les rainures longitudinales 17, en s'étendant selon une direction transversale orthogonale à la direction longitudinale X, et ce trou transversal 18 traverse la section centrale d'ancrage 14 pour former un chas de passage pour un ou plusieurs fils de suture 3, 4. Ainsi, le trou transversal 18 débouche dans les deux rainures longitudinales 17. Ce trou transversal 18 est ménagé au moins partiellement au niveau du cran distal de la succession de crans périphériques d'ancrage 16, et ce trou transversal 18 s'étend également sur la portion non crantée 140.

De plus, un pont 180 est prévu à l'intérieur du trou transversal 18 pour séparer deux passages 181, 182 décalés l'un de l'autre selon l'axe longitudinal X, avec un premier passage 181 situé au niveau du cran distal et un second passage 182 situé au niveau de la portion non crantée 140.

Il est en outre à noter que chaque rainure longitudinale 17 est prolongée, au niveau de la section distale d'insertion 13 et dans la continuité du trou transversal 18, par une rampe 19 en surélévation. Chaque rampe 19 est donc située entre la première extrémité distale 11 et le second passage 182, en alignement longitudinal avec la rainure longitudinale 17 correspondante, et cette rampe 19 forme une saillie par rapport à la portion non crantée 140, dont la fonction est de protéger un fil de suture extérieur 4 qui sera décrit ultérieurement.

En outre, le premier élément 1 présente un premier orifice longitudinal 100 qui est ménagé intérieurement dans la section proximale d'accouplement 15 et dans la section centrale d'ancrage 14 le long de l'axe longitudinal X. Ce premier orifice longitudinal 100 débouche d'un côté sur la première extrémité proximale 12 et débouche d'un autre côté sur le trou transversal 18 en face du pont 180. Aussi, ce premier orifice longitudinal 100 débouche sur le premier passage 181.

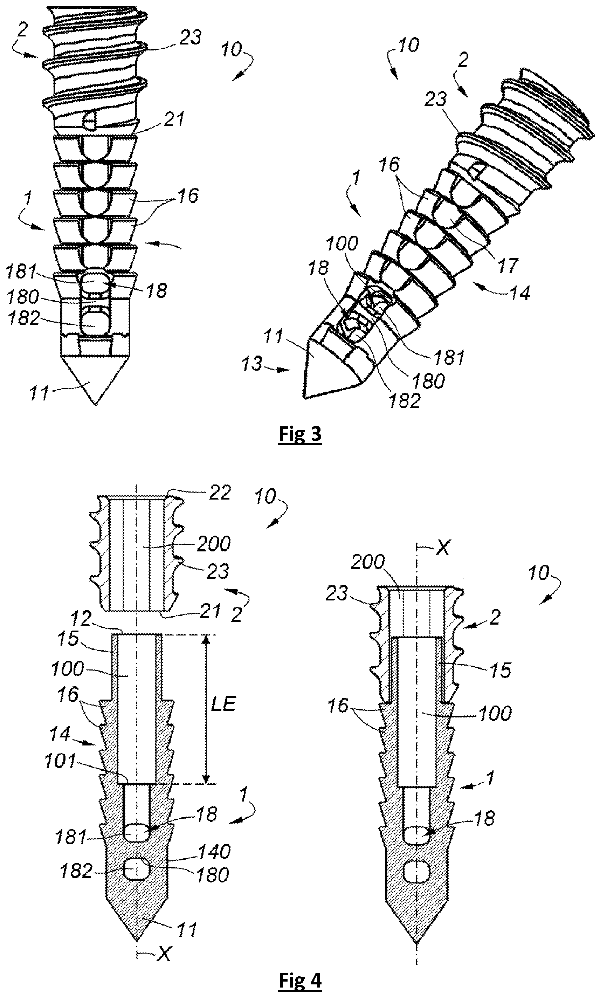

Par ailleurs, ce premier orifice longitudinal 100 peut être de section circulaire, et il présente un épaulement interne 101 correspondant à une réduction du diamètre interne de ce premier orifice longitudinal 100 en partant de la première extrémité proximale 12 en direction du trou transversal 18. Autrement dit, ce premier orifice longitudinal 100 présente une portion élargie (de plus grand diamètre) depuis la première extrémité proximale 12 jusqu'à l'épaulement interne 101, puis une portion rétrécie (de plus petit diamètre) depuis l'épaulement interne 101 jusqu'au trou transversal 18. En référence à la Figure 4, cette portion élargie du premier orifice longitudinal 100 présente une longueur LE mesurée le long de l'axe longitudinal X.

Le second élément 2 est indépendant du premier élément 1, dans le sens où il peut être complètement séparé et désaccouplé de ce premier élément 1. Ce second élément 2 présente le long de l'axe longitudinal X une seconde extrémité proximale 22 et une seconde extrémité distale 21 opposées, et il présente en outre un second orifice longitudinal 200 qui est ménagé intérieurement dans le second élément 2 le long de l'axe longitudinal X et qui débouche sur la seconde extrémité proximale 22 et sur la seconde extrémité distale 21. Ainsi, ce second élément 2 présente une forme générale de pièce tubulaire ou de manchon.

Ce second orifice longitudinal 200 est conformé pour pouvoir engager le second élément 2 sur la section proximale d'accouplement 15 du premier élément 1 avec la section proximale d'accouplement 15 à l'intérieur du second orifice longitudinal 200. Autrement dit, lorsque le premier élément 1 et le second élément 2 sont accouplés, le premier élément 1 a sa section proximale d'accouplement 15 qui est engagé à l'intérieur du second orifice longitudinal 200. Il est à noter que le second élément 2 est plus long que la section proximale d'accouplement 15, de sorte que lorsqu'ils sont accouplés, le premier élément 1 ne dépasse de la seconde extrémité proximale 22.

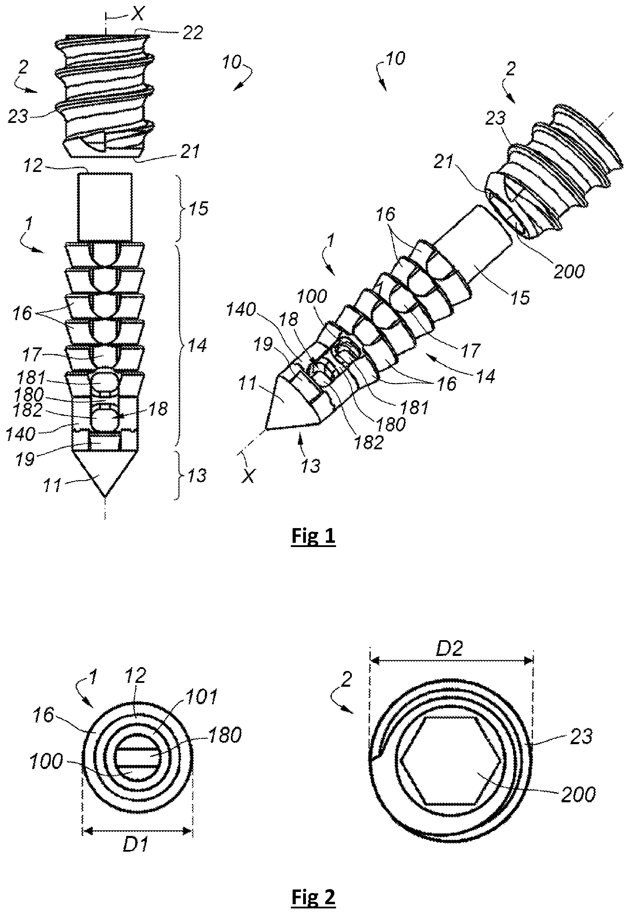

Ce second orifice longitudinal 200 est de section non circulaire, et par exemple de section polygonale comme une section hexagonale, comme visible en Figure 2. Ce second orifice longitudinal 200 et la section proximale d'accouplement 15 du premier élément 1 sont conformés pour que le second élément 2 soit libre en rotation autour de l'axe longitudinal X lorsque le second élément 2 est engagé sur la section proximale d'accouplement 15, autrement dit lorsque le premier élément 1 et le second élément 2 sont accouplés.

Le second élément 2 présente extérieurement un relief d'ancrage 23, et ce relief d'ancrage 23 prévu extérieurement sur le second élément 2 est un filetage. Ce relief d'ancrage 23 présente un second diamètre externe d'ancrage D2 qui est supérieur au premier diamètre externe d'ancrage D1. Avec un relief d'ancrage 23 sous forme d'un filetage, ce second diamètre externe d'ancrage D2 correspond au diamètre externe de filetage. Il est à noter qu'un tel filetage peut être avec un pas de vis évolutif pour permettre un verrouillage progressif du second élément 2 dans l'os 9.

La suite de la description porte sur un kit de suture comprenant une telle ancre de suture 10, ainsi qu'au moins un fil de suture 3, 4.

Dans une première réalisation, l'au moins un fil de suture comprend un fil de suture intérieur 3 qui passe dans le second orifice longitudinal 200 du second élément 2 et dans le premier orifice longitudinal 100 du premier élément 1 pour déboucher dans le trou transversal 18 et présenter une boucle 30 autour du pont 180, ce fil de suture intérieur 3 ayant ainsi deux brins libres 31 qui dépassent de la seconde extrémité proximale 22 du second élément 2 en débouchant hors du second orifice longitudinal 200.

Ainsi, lorsque l'ancre de suture 10 est ancré dans l'os 9, ce fil de suture intérieur 3 est libre de coulisser, en glissant sur le pont 180. De la sorte, un tel fil de suture intérieur 3 est ancré dans l'os 9 tout en conservant une mobilité, autrement dit ce fil de suture intérieur 3 peut coulisser en tirant sur l'un ou l'autre de ses brins libres 31.

Dans une deuxième réalisation, l'au moins un fil de suture comprend un fil de suture comprend un fil de suture extérieur 4 qui passe dans au moins une rainure longitudinale 17, sans passer dessus les crans périphériques d'ancrage 16, pour pénétrer dans le trou transversal 18 et présenter une boucle 40 autour du pont 180, ce fil de suture extérieur 4 ayant deux brins libres 41 qui circulent dans au moins une rainure longitudinale 17 et qui dépassent de la première extrémité proximale 12 du premier élément 1, en sortie de l'au moins une rainure longitudinale 17, pour être, en situation, bloqués par le relief d'ancrage 23 prévu sur l'extérieur du second élément 2. En situation, ces deux brins libres 41 dépassent de la seconde extrémité proximale 22 du second élément 2, en restant à l'extérieur de ce second élément 2.

Ainsi, lorsque l'ancre de suture 10 est ancré dans l'os 9, ce fil de suture extérieur 4 reste fixe, en étant coincé entre l'os 9 et le relief d'ancrage 23 du second élément 2. De la sorte, un tel fil de suture extérieur 4 est ancré dans l'os 9 et il ne peut pas coulisser et reste statique quand bien même on tirerait sur l'un ou l'autre de ses brins libres 41. Il est envisageable que les deux brins libres 41 du fil de suture extérieur 4 passent dans une même rainure longitudinale 17 ou, en variante, passent dans respectivement dans les deux rainures longitudinale 17 opposées.

Il est à noter que l'ancre de suture 10 peut être associée à un ou plusieurs fils de suture intérieur 3 et/ou à un ou plusieurs fils de suture extérieur 4.

Le kit de suture peut également comprendre un instrument d'insertion 5 propre à insérer par impaction le premier élément 1 de l'ancre de suture 10, où cet instrument d'insertion 5 présente un orifice interne longitudinal 50 autorisant le passage d'un ou plusieurs fils de suture intérieur 3. Autrement dit, cet instrument d'insertion 5 présente une forme générale de pièce tubulaire.

Cet instrument d'insertion 5 présente une partie distale 51 adaptée pour être engagée à l'intérieur du premier orifice longitudinal 100 du premier élément 1, et cette partie distale 51 est de forme cylindrique et conformée pour être libre en rotation autour de l'axe longitudinal X à l'intérieur du premier orifice longitudinal 100. Cette partie distale 51 peut présenter un diamètre externe qui est, au jeu de montage près, équivalent au diamètre interne de la portion élargie de ce premier orifice longitudinal 100, de sorte que cette partie distale 51 est montée ajustée à l'intérieur de cette portion élargie du premier orifice longitudinal 100.

Par ailleurs, la partie distale 51 de l'instrument d'insertion 5 présente une extrémité distale 53 venant en appui sur l'épaulement interne 101 du premier orifice longitudinal 100, où cette partie distale 51 dépasse de la première extrémité proximale 12 du premier élément 1 afin que cet instrument d'insertion 5 ne vienne pas en appui contre cette première extrémité proximale 12. Autrement dit, cette partie distale 51 présente une longueur qui est supérieure à la longueur LE de la portion élargie du premier orifice longitudinal 100. Ainsi, l'effort d'impaction procuré par l'instrument d'insertion 5 sur le premier élément 1 se fait au niveau de l'extrémité distale 53 de l'instrument d'insertion 5 en appui sur l'épaulement interne 101 du premier orifice longitudinal 100.

L'instrument d'insertion 5 présente également, dans le prolongement de la partie distale 51, une partie proximale 52 autour de laquelle peut être monté coulissant le second élément 2, cette partie proximale 52 traversant le second orifice longitudinal 200 du second élément 2. Cette partie proximale 52 de l'instrument d'insertion 5 est de section complémentaire à celle du second orifice longitudinal 200, de sorte que le second élément 2 est solidaire en rotation de l'instrument d'insertion 5 autour de l'axe longitudinal X. Cette partie proximale 52 permet ainsi de faire tourner le second élément 2 lors de son insertion, pour une insertion par vissage dans l'os 9.

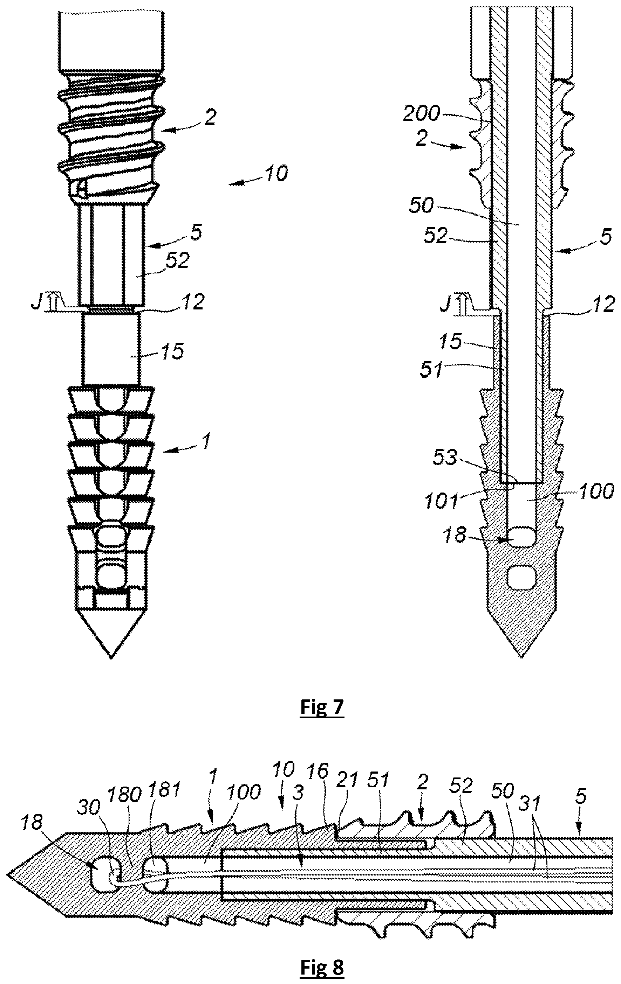

Cette partie proximale 52 présente une dimension transversale supérieure au diamètre externe de la partie distale 51, et cette partie proximale 52 va, en situation, présenter être espacé avec un jeu J de la première extrémité proximale 12 du premier élément 1, comme illustré sur la Figure 7.

La suite de la description porte sur l'utilisation d'une telle ancre de suture 10, et en particulier sur l'ancrage du ou des fils de suture 1 dans l'os 9.

Dans une première étape :
- le fil de suture intérieur 3 est préalablement engagé dans le premier orifice longitudinal 100 du premier élément 1, avec sa boucle 30 autour du pont 180, et avec ses deux brins libres 31 qui dépassent de la première extrémité proximale 12 du premier élément 1 ; et/ou
- le fil de suture extérieur 4 est préalablement engagé dans au moins une rainure longitudinale 17 du premier élément 1, avec sa boucle 40 autour du pont 180, et avec ses deux brins libres 31 qui dépassent de la première extrémité proximale 12.

Dans une seconde étape, le second élément 2 est engagé autour de la partie proximale 52 de l'instrument d'insertion 5, puis la partie distale 51 de l'instrument d'insertion 5 est engagé à l'intérieur du premier orifice longitudinal 100, en prenant soin que les brins libres 31 du fil de suture intérieur 3 passent à l'intérieur de l'instrument d'insertion 5, dans son orifice interne longitudinal 50 comme visible en Figure 8, et que les brins libres 41 du fil de suture extérieur 4 passent dans au moins une rainure longitudinale 17. A ce stade, le second élément 2 est à distance du premier élément 1.

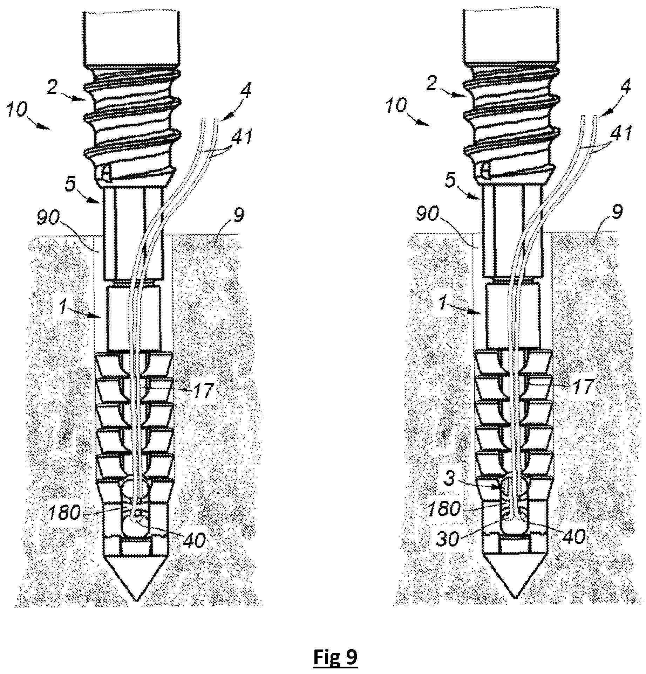

Dans une troisième étape, le premier élément 1 est inséré par impaction dans l'os 9, au moyen de l'instrument d'insertion 5, à l'intérieur d'un trou 90 dans l'os 9. Il est envisageable que ce trou 90 soit au moins partiellement perforé à l'avance et/ou ce trou 90 est ménagé lors d'impaction grâce à la première extrémité distale 11 qui forme une extrémité auto-perforante. Le premier élément 1 est inséré suffisamment profond pour laisser la place au second élément 2 qui, à ce stade, est encore à l'extérieur du trou 90, comme visible en Figure 9. Le fil de suture extérieur 4 peut encore être coulissé et ajusté, avant l'ancrage finale.

Dans une quatrième étape, le second élément 2 est inséré par vissage, au moyen de l'instrument d'insertion 5 qui est tourné autour de l'axe longitudinal X, de sorte que le second élément 2 se visse dans le trou 90 jusqu'à ce que sa seconde extrémité distale 21 vienne contre le cran proximal de la succession de crans périphériques d'ancrage 16 (comme illustré en Figure 8), et vient ainsi finaliser et bloquer l'ancre de suture 10 dans le trou 90, tout en coinçant le fil de suture extérieur 4 qui ne pourra alors plus être ajusté, alors que le fil de suture intérieur 3 reste ajustable.

## Revendications

1. Ancre de suture (10), pour un ancrage d'un tissu mou sur un os (9), ladite ancre de suture (10) comprenant un premier élément (1) présentant le long d'un axe longitudinal (X) une première extrémité distale (11) et une première extrémité proximale (12) opposées, ledit premier élément (1) présentant successivement, depuis la première extrémité distale (11) jusqu'à la première extrémité proximale (12), une section distale d'insertion (13), une section centrale d'ancrage (14) et une section proximale d'accouplement (15), où la section centrale d'ancrage (14) présente extérieurement et le long de l'axe longitudinal (X) une succession de crans périphériques d'ancrage (16), où au moins une rainure longitudinale (17) s'étend le long de l'axe longitudinal (X) et est ménagée à travers et le long de la succession de crans périphériques d'ancrage (16), et où un trou transversal (18) est ménagé dans ladite rainure longitudinale (17) et traverse la section centrale d'ancrage (14) pour former un chas de passage de fil de suture (3 ; 4), et dans lequel un premier orifice longitudinal (100) est ménagé intérieurement dans la section proximale d'accouplement (15) et dans la section centrale d'ancrage (14) le long de l'axe longitudinal (X), ledit premier orifice longitudinal (100) débouchant sur la première extrémité proximale (12) et débouchant sur le trou transversal (18) ;
ladite ancre de suture (10) comprenant en outre un second élément (2) indépendant du premier élément (1), ledit second élément (2) présentant une seconde extrémité proximale (22) et une seconde extrémité distale (21) opposées, où le second élément (2) présente extérieurement au moins un relief d'ancrage (23), et où un second orifice longitudinal (200) est ménagé intérieurement dans le second élément (2) et débouche sur la seconde extrémité proximale (22) et sur la seconde extrémité distale (21), ledit second orifice longitudinal (200) étant conformé pour pouvoir engager le second élément (2) sur la section proximale d'accouplement (15) du premier élément (1) avec ladite section proximale d'accouplement (15) à l'intérieur dudit second orifice longitudinal (200) ;
ladite ancre de suture (10) étant **caractérisée en ce qu'**un pont (180) est prévu à l'intérieur du trou transversal (18) pour séparer deux passages (181, 182) décalés l'un de l'autre selon l'axe longitudinal (X), et **en ce que** ledit premier orifice longitudinal (100) débouche sur le trou transversal (18) en face du pont (180).

2. Ancre de suture (10) selon l'une quelconque des revendications précédentes, dans laquelle deux rainures longitudinales (17) sont ménagées à travers et le long de la succession de crans périphériques d'ancrage (16), les deux rainures longitudinales (17) étant diamétralement opposées vis-à-vis de l'axe longitudinal (X) de sorte que le trou transversal (18) débouche dans les deux rainures longitudinales (17).

3. Ancre de suture (10) selon l'une quelconque des revendications précédentes, dans laquelle l'au moins une rainure longitudinale (17) est prolongée, au niveau de la section distale d'insertion (13) et dans la continuité du trou transversal (18), par une rampe (19) en surélévation.

4. Ancre de suture (10) selon l'une quelconque des revendications précédentes, dans laquelle la première extrémité distale (11) est en forme de pointe.

5. Ancre de suture (10) selon l'une quelconque des revendications précédentes, dans laquelle le second orifice longitudinal (200) est de section non circulaire, et par exemple de section polygonale comme une section hexagonale.

6. Ancre de suture (10) selon l'une quelconque des revendications précédentes, dans laquelle le second orifice longitudinal (200) et la section proximale d'accouplement (15) sont conformés pour que le second élément (2) soit libre en rotation autour de l'axe longitudinal (X) lorsque ledit second élément (2) est engagé sur la section proximale d'accouplement (15).

7. Ancre de suture (10) selon l'une quelconque des revendications précédentes, dans laquelle le premier orifice longitudinal (100) présente un épaulement interne (101).

8. Kit de suture, pour un ancrage d'un tissu mou sur un os (9), ledit kit de suture comprenant une ancre de suture (10) selon l'une quelconque des revendications précédentes, et au moins un fil de suture (3 ; 4) formant une boucle (30 ; 40) sur le pont (180) prévu dans le trou transversal (18).

9. Kit de suture selon la revendication 8, dans lequel l'au moins un fil de suture comprend un fil de suture intérieur (3) qui passe dans le second orifice longitudinal (200) du second élément (2) et dans le premier orifice longitudinal (100) du premier élément (1) pour déboucher dans le trou transversal (18) et présenter une boucle (30) autour du pont (180), ledit fil de suture intérieur (3) ayant deux brins libres (31) qui dépassent de la seconde extrémité proximale (22) du second élément (2).

10. Kit de suture selon les revendications 8 ou 9, dans lequel l'au moins un fil de suture comprend un fil de suture extérieur (4) qui passe dans l'au moins une rainure longitudinale (17), sans passer dessus les crans périphériques d'ancrage (16), pour pénétrer dans le trou transversal (18) et présenter une boucle (40) autour du pont (180), ledit fil de suture extérieur (4) ayant deux brins libres (41) qui dépassent de la première extrémité proximale (12) du premier élément (1), en sortie de l'au moins une rainure longitudinale (17), pour être, en situation, bloqués par l'au moins un relief d'ancrage (23) prévu sur l'extérieur du second élément (2).

11. Kit de suture selon l'une quelconque des revendications 8 à 10, comprenant en outre un instrument d'insertion (5) propre à insérer par impaction le premier élément (1) de l'ancre de suture (10), ledit instrument d'insertion (5) présentant un orifice interne longitudinal (50) autorisant le passage d'au moins un fil de suture (3) et présentant une partie distale (51) engagée à l'intérieur du premier orifice longitudinal (100) du premier élément (1).

12. Kit de suture selon la revendication 11, dans lequel l'ancre de suture (10) est conforme à la revendication 7 de sorte que le premier orifice longitudinal (100) présente l'épaulement interne (101), et la partie distale (51) de l'instrument d'insertion (5) présente une extrémité distale (53) venant en appui sur ledit épaulement interne (101), où la partie distale (51) dépasse de la première extrémité proximale (12) du premier élément (1) de sorte que ledit instrument d'insertion (5) ne vient pas en appui contre ladite première extrémité proximale (12).

13. Kit de suture selon les revendications 11 ou 12, dans lequel la partie distale (51) de l'instrument d'insertion (5) est libre en rotation autour de l'axe longitudinal (X) à l'intérieur du premier orifice longitudinal (100).

14. Kit de suture selon l'une quelconque des revendications 11 à 13, dans lequel l'instrument d'insertion (5) présente, dans le prolongement de la partie distale (51), une partie proximale (52) autour de laquelle est monté coulissant le second élément (2), ladite partie proximale (52) traversant le second orifice longitudinal (200).

15. Kit de suture selon la revendication 14, dans lequel le second orifice longitudinal (200) est de section non circulaire, et par exemple de section polygonale comme une section hexagonale, et la partie proximale (52) de l'instrument d'insertion (5) est de section complémentaire à celle du second orifice longitudinal (200), de sorte que le second élément (2) est solidaire en rotation de l'instrument d'insertion (5) autour de l'axe longitudinal (X).

## Patentansprüche

1. Nahtanker (10) zur Verankerung von Weichgewebe an einem Knochen (9), wobei der Nahtanker (10) ein erstes Element (1) umfasst, das entlang einer Längsachse (X) ein erstes gegenüberliegendes distales Ende (11) und ein erstes proximales Ende (12) aufweist, wobei das erste Element (1) von dem ersten distalen Ende (11) bis zum ersten proximalen Ende (12) nacheinander einen distalen Einführabschnitt (13), einen mittleren Ankerabschnitt (14) und einen proximalen Koppelabschnitt (15) aufweist, wobei der mittlere Ankerabschnitt (14) außen und entlang der Längsachse (X) eine Abfolge von umlaufenden Ankerkerben (16) aufweist, wobei sich mindestens eine Längsnut (17) entlang der Längsachse (X) erstreckt und durch und entlang der Abfolge von umlaufenden Ankerkerben (16) ausgebildet ist, und wobei ein Querloch (18) in der Längsnut (17) und durch den mittleren Ankerabschnitt (14) ausgebildet ist, um ein Nahtfadendurchführungsöhr (3; 4) zu bilden, und wobei ein erstes Längsloch (100) innen im proximalen Koppelabschnitt (15) und im mittleren Ankerabschnitt (14) entlang der Längsachse (X) ausgebildet ist, wobei das erste Längsloch (100) am ersten proximalen Ende (12) mündet und am Querloch (18) mündet;
wobei der Nahtanker (10) ferner ein zweites Element (2) umfasst, das unabhängig vom ersten Element (1) ist, wobei das zweite Element (2) ein gegenüberliegendes zweites proximales Ende (22) und ein zweites distales Ende (21) aufweist, wo das zweite Element (2) außen mindestens eine Verankerungserhebung (23) aufweist, und wo ein zweites Längsloch (200) innen im zweiten Element (2) vorgesehen ist und am zweiten proximalen Ende (22) und am zweiten distalen Ende (21) mündet, wobei das zweite Längsloch (200) so ausgebildet ist, dass das zweite Element (2) auf den proximalen Koppelabschnitt (15) des ersten Elements (1) mit dem proximalen Koppelabschnitt (15) innerhalb des zweiten Längslochs (200) eingreifen kann;
wobei der Nahtanker (10) **dadurch gekennzeichnet ist, dass** innerhalb des Querlochs (18) eine Brücke (180) vorgesehen ist, um zwei in der Längsachse (X) versetzt voneinander angeordnete Durchgänge (181, 182) zu trennen, und, dass das erste Längsloch (100) auf das Querloch (18) gegenüber der Brücke (180) mündet.

2. Nahtanker (10) nach einem der vorhergehenden Ansprüche, wobei zwei Längsnuten (17) durch und entlang der Abfolge von umlaufenden Ankerkerben (16) vorgesehen sind, wobei die beiden Längsnuten (17) sich diametral gegenüber der Längsachse (X) befinden, so dass das Querloch (18) in die beiden Längsnuten (17) mündet.

3. Nahtanker (10) nach einem der vorhergehenden Ansprüche, wobei die mindestens eine Längsnut (17) am distalen Einführabschnitt (13) und in der Kontinuität des Querlochs (18) durch eine Rampe (19) in Erhöhung verlängert ist.

4. Nahtanker (10) nach einem der vorhergehenden Ansprüche, wobei das erste distale Ende (11) spitzenförmig ist.

5. Nahtanker (10) nach einem der vorhergehenden Ansprüche, wobei das zweite Längsloch (200) einen unrunden Querschnitt, und beispielsweise einen polygonalen Querschnitt wie einen sechseckigen Querschnitt aufweist.

6. Nahtanker (10) nach einem der vorhergehenden Ansprüche, wobei das zweite Längsloch (200) und der proximale Koppelabschnitt (15) so ausgebildet sind, dass das zweite Element (2) frei um die Längsachse (X) rotieren kann, wenn das zweite Element (2) auf den proximalen Koppelabschnitt (15) eingreift.

7. Nahtanker (10) nach einem der vorhergehenden Ansprüche, wobei das erste Längsloch (100) eine Innenschulter (101) aufweist.

8. Nahtset zur Verankerung von Weichgewebe an einem Knochen (9), wobei das Nahtset einen Nahtanker (10) nach einem der vorhergehenden Ansprüche und mindestens einen Nahtfaden (3; 4) umfasst, der eine Schlaufe (30; 40) an der in dem Querloch (18) vorgesehenen Brücke (180) bildet.

9. Nahtset nach Anspruch 8, wobei der mindestens eine Nahtfaden einen inneren Nahtfaden (3) umfasst, der durch das zweite Längsloch (200) des zweiten Elements (2) und durch das erste Längsloch (100) des ersten Elements (1) führt, um in das Querloch (18) zu münden und eine Schlaufe (30) um die Brücke (180) aufzuweisen, wobei der innere Nahtfaden (3) zwei freie Stränge (31) aufweist, die aus dem zweiten proximalen Ende (22) des zweiten Elements (2) herausragen.

10. Nahtset nach Anspruch 8 oder 9, wobei der mindestens eine Nahtfaden einen äußeren Nahtfaden (4) umfasst, der in die mindestens eine Längsnut (17) verläuft, ohne über die umlaufenden Ankerkerben (16) zu verlaufen, um in das Querloch (18) einzudringen und eine Schlaufe (40) um die Brücke (180) aufzuweisen, wobei der äußere Nahtfaden (4) zwei freie Stränge (41) aufweist, die aus dem ersten proximalen Ende (12) des ersten Elements (1) herausragen, am Ausgang der mindestens einen Längsnut (17), um in dieser Situation durch die mindestens eine Verankerungserhebung (23) gesperrt zu werden, die an der Außenseite des zweiten Elements (2) vorgesehen ist.

11. Nahtset nach einem der Ansprüche 8 bis 10, ferner umfassend ein Einführinstrument (5), das geeignet ist, das erste Element (1) des Nahtankers (10) einzuschlagen, wobei das Einführinstrument (5) ein Längsinnenloch (50) aufweist, das das Durchführen von mindestens einem Nahtfaden (3) ermöglicht, und einen distalen Teil (51) aufweist, der in das erste Längsloch (100) des ersten Elements (1) eingreift.

12. Nahtset nach Anspruch 11, wobei der Nahtanker (10) nach Anspruch 7 so ausgelegt ist, dass das erste Längsloch (100) die Innenschulter (101) aufweist, und der distale Teil (51) des Einführinstruments (5) ein distales Ende (53) aufweist, das auf der Innenschulter (101) aufliegt, wo der distale Teil (51) aus dem ersten proximalen Ende (12) des ersten Elements (1) herausragt, so dass das Einführinstrument (5) nicht an dem ersten proximalen Ende (12) anliegt.

13. Nahtset nach Anspruch 11 oder 12, wobei der distale Teil (51) des Einführinstruments (5) frei um die Längsachse (X) innerhalb des ersten Längslochs (100) drehbar ist.

14. Nahtset nach einem der Ansprüche 11 bis 13, wobei das Einführinstrument (5) in Verlängerung des distalen Teils (51) einen proximalen Teil (52) aufweist, um den das zweite Element (2) gleitend montiert ist, wobei der proximale Teil (52) durch das zweite Längsloch (200) geht.

15. Nahtset nach Anspruch 14, wobei das zweite Längsloch (200) einen unrunden Querschnitt, und beispielsweise einen polygonalen Querschnitt wie einen Sechskantquerschnitt hat, und der proximale Teil (52) des Einführinstruments (5) einen Querschnitt hat, der zu dem des zweiten Längslochs (200) komplementär ist, so dass das zweite Element (2) um die Längsachse (X) drehfest mit dem Einführinstrument (5) verbunden ist.

## Claims

1. Suture anchor (10), for anchoring soft tissue to a bone (9), said suture anchor (10) comprising a first element (1) having along a longitudinal axis (X) a first distal end (11) and a first proximal end (12) opposite each other, said first element (1) having successively, from the first distal end (11) to the first proximal end (12), a distal insertion section (13), a central anchoring section (14) and a proximal coupling section (15), where the central anchoring section (14) has externally and along the longitudinal axis (X) a succession of peripheral anchoring notches (16), wherein at least one longitudinal groove (17) extends along the longitudinal axis (X) and is arranged through and along the succession of peripheral anchoring notches (16), and wherein a transverse hole (18) is provided in said longitudinal groove (17) and passes through the central anchoring section (14) to form an eye for the passage of a suture thread (3; 4), and wherein a first longitudinal orifice (100) is provided internally in the proximal coupling section (15) and in the central anchoring section (14) along the longitudinal axis (X), said first longitudinal orifice (100) opening onto the first proximal end (12) and opening onto the transverse hole (18);
said suture anchor (10) further comprising a second element (2) independent of the first element (1), said second element (2) having a second proximal end (22) and a second distal end (21) opposite each other, wherein the second element (2) has externally at least one anchoring relief (23), and wherein a second longitudinal orifice (200) is arranged internally in the second element (2) and opens onto the second proximal end (22) and onto the second distal end (21), said second longitudinal orifice (200) being shaped to be able to engage the second element (2) on the proximal coupling section (15) of the first element (1) with said proximal coupling section (15) inside said second longitudinal orifice (200);
said suture anchor (10) being **characterized in that** a bridge (180) is provided inside the transverse hole (18) to separate two passages (181, 182) offset from each other along the longitudinal axis (X), and **in that** said first longitudinal orifice (100) opens onto the transverse hole (18) in front of the bridge (180).

2. Suture anchor (10) according to any one of the preceding claims, wherein two longitudinal grooves (17) are provided through and along the succession of peripheral anchoring notches (16), the two longitudinal grooves (17) being diametrically opposed with respect to the longitudinal axis (X) so that the transverse hole (18) opens into the two longitudinal grooves (17).

3. Suture anchor (10) according to any one of the preceding claims, wherein the at least one longitudinal groove (17) is extended, at the distal insertion section (13) and in continuity with the transverse hole (18), by a raised ramp (19).

4. Suture anchor (10) according to any one of the preceding claims, wherein the first distal end (11) is spike shaped.

5. Suture anchor (10) according to any one of the preceding claims, wherein the second longitudinal orifice (200) has a non-circular section, and for example a polygonal section such as a hexagonal section.

6. Suture anchor (10) according to any one of the preceding claims, wherein the second longitudinal orifice (200) and the proximal coupling section (15) are shaped so that the second element (2) is free to rotate around the longitudinal axis (X) when said second element (2) is engaged on the proximal coupling section (15).

7. Suture anchor (10) according to any one of the preceding claims, wherein the first longitudinal orifice (100) has an internal shoulder (101).

8. Suture kit, for anchoring soft tissue to a bone (9), said suture kit comprising a suture anchor (10) according to any one of the preceding claims, and at least one suture thread (3; 4) forming a loop (30; 40) on the bridge (180) provided in the transverse hole (18).

9. Suture kit according to claim 8, wherein the at least one suture thread comprises an inner suture thread (3) which passes through the second longitudinal orifice (200) of the second element (2) and through the first longitudinal orifice (100) of the first element (1) to arrive into the transverse hole (18) and present a loop (30) around the bridge (180), said inner suture thread (3) having two free strands (31) which protrude from the second proximal end (22) of the second element (2).

10. Suture kit according to claims 8 or 9, wherein the at least one suture thread comprises an outer suture thread (4) which passes into the at least one longitudinal groove (17), without passing over the peripheral anchoring notches (16), to penetrate into the transverse hole (18) and present a loop (40) around the bridge (180), said outer suture thread (4) having two free strands (41) which protrude from the first proximal end (12) of the first element (1), at the outlet of the at least one longitudinal groove (17), to be, in situation, blocked by the at least one anchoring relief (23) provided on the outside of the second element (2).

11. Suture kit according to any one of claims 8 to 10, further comprising an insertion instrument (5) suitable for inserting by impaction the first element (1) of the suture anchor (10), said insertion instrument (5) having a longitudinal internal orifice (50) allowing the passage of at least one suture thread (3) and having a distal part (51) engaged inside the first longitudinal orifice (100) of the first element (1).

12. Suture kit according to claim 11, wherein the suture anchor (10) is according to claim 7 such that the first longitudinal orifice (100) has the internal shoulder (101), and the distal portion (51) of the insertion instrument (5) has a distal end (53) bearing on said internal shoulder (101), wherein the distal portion (51) protrudes from the first proximal end (12) of the first element (1) such that said insertion instrument (5) does not bear against said first proximal end (12).

13. Suture kit according to claims 11 or 12, wherein the distal portion (51) of the insertion instrument (5) is free to rotate around the longitudinal axis (X) inside the first longitudinal orifice (100).

14. Suture kit according to any one of claims 11 to 13, wherein the insertion instrument (5) has, in the extension of the distal part (51), a proximal part (52) around which the second element (2) is slidably mounted, said proximal part (52) passing through the second longitudinal orifice (200).

15. Suture kit according to claim 14, wherein the second longitudinal orifice (200) has a non-circular section, and for example a polygonal section such as a hexagonal section, and the proximal part (52) of the insertion instrument (5) has a section complementary to that of the second longitudinal orifice (200), so that the second element (2) is secured in rotation to the insertion instrument (5) around the longitudinal axis (X).
